# EUROPEAN PATENT SPECIFICATION

(11) **EP 1 281 764 B1**
(45) Date of publication and mention of the grant of the patent: **03.11.2010**
(21) Application number: 02255169.1
(22) Date of filing: 24.07.2002
(51) Int. Cl.: C12N 15/82

(54) **Method for introducing gene into plant having improved transformation efficiency.**
Verfahren zur Einführung eines Gens in Pflanzen mit Verbesserung der Transformationeffizienz
Methode pour l'introduction d'un gène dans une plante avec amelioration de l'efficacité de transformation

(30) Priority: 24.07.2001 JP 2001223664
(43) Date of publication of application: 05.02.2003
(73) Proprietor: NIPPON PAPER INDUSTRIES CO., LTD., Kita-ku, Tokyo (JP)
(72) Inventor: Matsunaga, Etsuko, c/o Nippon Paper Ind. Co. Ltd., Tokyo (JP); Sugita, Koichi, c/o Nippon Paper Ind. Co. Ltd., Tokyo (JP); Ebinuma, Hiroyasu, c/o Nippon Paper Ind. Co. Ltd., Tokyo (JP)
(74) Representative: Smyth, Gyles Darren

(56) References cited:
- EP-A- 1 050 209
- WO-A-01/42480
- WO-A-01/53452
- WO-A-94/02620
- WO-A-97/25434
- BRASILEIRO A C M ET AL: "AN ALTERNATIVE APPROACH FOR GENE TRANSFER IN TREES USING WILD-TYPE AGROBACTERIUM STRAINS" PLANT MOLECULAR BIOLOGY, NIJHOFF PUBLISHERS, DORDRECHT, NL, vol. 17, 1991, pages 441-452, XP002030459 ISSN: 0167-4412
- PATENT ABSTRACTS OF JAPAN vol. 2002, no. 02, 2 April 2002 (2002-04-02) & JP 2001 275667 A (NIPPON PAPER INDUSTRIES CO LTD), 9 October 2001 (2001-10-09)
- PATENT ABSTRACTS OF JAPAN vol. 2002, no. 02, 2 April 2002 (2002-04-02) & JP 2001 275668 A (NIPPON PAPER INDUSTRIES CO LTD), 9 October 2001 (2001-10-09)
- GOULD J. , DEVEY M., HASEGAWA O. ET AL.: "Transformation of Zea mays L. using Agrobacterium tumefaciens and the shoot apex" PLANT PHYSIOL., vol. 95, February 1991 (1991-02), pages 426-434, XP002179154
- BIOLOGICAL ABSTRACTS, no. P270, January 1995 (1995-01), Philadelphia, PA, US, GOULD J.: "Shoot apex transformation of Loblolly Pine" page 1 XP002242924
- MANTE S., MORGENS P.H., SCORZA R., ET AL.: "Agrobacterium.mediated transformation of plum (Prunus Domestica L.) hypocotyl slices and regeneration of transgenic plants." BIOTECHNOLOGY, vol. 9, 1991, pages 853-857,

## Description

### BACKGROUND OF THE INVENTION

### 1. Field of the Invention

The present invention relates to a method for improving transformation efficiency in the introduction of a desired gene into a plant using genetic engineering techniques.

### 2. Description of the Background

According to gene introduction into a plant using genetic engineering techniques, a desired gene can be directly introduced into a plant. Accordingly, it has many advantages over the classical breeding which repeats crossing. For example, according to the gene introduction, (a) only a character to be modified can be introduced, (b) characters of species other than plants (microorganisms and the like) can also be introduced into a plant, and (c) a breeding period can be markedly reduced.

However, there are many plants in that the advantages cannot be obtained sufficiently depending on the kind of the tree due to low transformation efficiency by genetic engineering techniques. For example, since trees have a long life cycle, the conventional breeding which is carried out by repeating crossing requires a vast land and a long years. Also, trees are one of the most important biomass resources and expected in recent years to contribute to the maintenance and improvement of terrestrial environment, so that concern has been directed toward the development of varieties having more excellent properties such as productivity, environmental resistance and the like. Thus, although the breeding by gene introduction produces large advantages also in trees, the transformation efficiency into industrially important tree species is very low in most cases.

Accordingly, for transformation of such trees, attempts have been made to improve the transformation efficiency through examinations from various points of view, such as kinds of tissues to be used, conditions for the gene introduction treatment and culture conditions of the tissue before and after the treatment. A method in which a gene introduction treatment is carried out using a shoot as the tissue for gene introduction, and an adventitious bud is re-differentiated therefrom to obtain a desired gene-introduced individual, namely a transformant, has also been examined on tree species.

However, it is also difficult to improve the efficiency of tree species which have originally low transformation efficiency. After all, actually, an industrially important tree species whose properties have been improved to practically valuable levels by genetic engineering techniques has not been obtained yet.

Also, up to date, the methods already established as the preparation method of transformed plants are those which had been established by defining the gene introduction treatment conditions, culture conditions of tissues for gene introduction and the like, in response to the respective kinds of plants to be used, and attempting to improve the transformation efficiency individually. A transformation method which can improve the transformation efficiency common to a number of plants is also not known yet.

### SUMMARY OF THE INVENTION

An object to the present invention is to provide a method for producing transgenic plants applied to a number of plants including trees in which transformation is considered to be difficult, by improving transformation efficiency using genetic engineering techniques.

This and other objects of the present invention have been accomplished by a method for producing a transgenic plant, which comprises:
preparing a tissue for gene introduction by cutting off a root and a root base from a hypocotyl, obtained by germinating a seed, having an apical bud or an apical bud primordium, to thereby form an end face whilst retaining the apical bud of the apical bud primordium; and
introducing a desired gene into the base part of the hypocotyl, to thereby differentiate an adventitious bud.

EP1050209A discloses a method of transformation, wherein adventitious shoots obtained from the explant of *Eucalyptus* plants are used as the tissue for gene introduction. The adventitious shoots are shoots obtained from tissues which normally do not bud, under natural conditions. In this art, the adventitious shoots, such as precocious branches (that blossom before the appearance of leaves) or axillary shoots, obtained by tissue culture of tissues having a growing point of a plant, are used as the tissue for gene introduction. Thus, EP1050209A discloses tissues that do not normally give rise to buds, whilst we specify the hypocotyl having an apical bud or an apical bud primordium. In WO 97/25434A, the desired gene is introduced into the growing point of shoots. In Gould et al (Plant Physiol., vol 95, Feb 1991 pp426-434 and Biological Abstracts, Plant Genome Conference, Abstract no. P270, Jan 1995) the desired gene is introduced into the shoot apex. In contrast, in the present invention, the desired gene is introduced into the base part of the hypocotyl, such that the hypocotyl is the tissue for gene introduction. Mante et al (Biotechnology, vol. 9, 1991, p853-857) relates to a method for the regeneration of plum plants from the hypocotyl region of the embryonic axes of un-germinated seeds, the application of this protocol in Agrobacterium-mediated transformation, and the successful regeneration and establishment of transgenic plants in the greenhouse. In contrast, the present invention involves a hypocotyl obtained by germinating a seed.

### BRIEF DESCRIPTION OF THE DRAWINGS

Fig. 1 is a schematic view showing the structure of a T-DNA region which is incorporated into a plant gene in pBI121 vector.
Fig. 2 is a schematic view showing the structure of a T-DNA region which is incorporated into a plant gene in pIPT10 vector.

### DETAILED DESCRIPTION OF THE INVENTION

As a result of intensive studies, the present inventors have found that when a hypocotyl obtained by germinating a seed, where the root and a root base have been cut off from the hypocotyl, the hypocotyl having an apical bud or an apical bud primordium, to thereby form an end face whilst retaining the apical bud or the apical bud primordium, is used as a tissue for gene introduction and a desired gene is introduced into a base part of the hypocotyl, the desired gene is efficiently introduced into a cell of the base part and furthermore, the base part of the hypocotyl to which the desired gene has been thus introduced has high ability to redifferentiate a desired gene-introduced adventitious bud. Thus, the present invention has been accomplished.

The present invention can be applied to any plants regardless of their species. However, effects of the present invention can be particularly obtained in plants in which gene introduction is considered to be difficult, such as trees and the like.

In the present invention, the shoot to be used as the tissue for gene introduction means an elongated normal bud or adventitious bud. Also, in order to further improve the transformation efficiency, it is preferable to use, as the tissue for gene introduction, a tissue or part having high redifferentiation potency such as a young tissue having a low content of polyphenols which become the cause of browning of tissues. From this point of view, a hypocotyl obtained by germinating a seed is selected in the present invention as the tissue for gene introduction.

However, the shoot must have at least one end having a growing point and a base part having no growing point. As a hypocotyl is used as the tissue for gene introduction of the present invention, the shoot can be obtained by cutting off a root and a part having a base of root from a hypocotyl after germination of a seed while leaving the apical bud and apical bud primordium as they are. In this case, the one end having a growing point means a shoot end having an apical bud and the like, and the base part having no growing point means a part having the end face formed by cutting off a root and the like. That is, the desired gene is preferably introduced into the end face of the base part of the hypocotyl generated when a hypocotyl is prepared as the tissue for gene introduction.

In the present invention, an adventitious bud is differentiated by introducing a desired gene into the base part having no growing point, while keeping the apical bud our apical bud primordium. As the desired gene, various genes such as a gene which can provide an industrially excellent character and a gene which cannot always provide an industrially excellent character but is necessary in studying gene expression mechanism can be selected and used.

A desired gene can be introduced into the base part of the hypocotyl indirectly via Gemini virus, Brome mosaic virus, *Agrobacterium tumefaciens* (hereinafter referred to as "*A. tumefaciens*"), *Agrobacterium rhizogenes* and the like viruses and bacteria, by inserting the desired gene into an appropriate vector, or directly by the particle gun method and the like (I. Potrykus, Annu. Rev. Plant Physiol. Plant Mol. Biol., 42: 205 (1991)).

For example, in a gene introduction method using *Agrobacterium* (*Agrobacterium* method, e.g., EP-A-0290799, U.S. Patents 4,940,838 and 5,591,616 and the like), an appropriate vector into which a desired gene has been inserted is introduced into *Agrobacterium* in advance, and the desired gene is introduced into a tissue for gene introduction by infection with the *Agrobacterium.* The infection of the *Agrobacterium* is carried out, for example, by soaking the tissue for gene introduction in a solution in which the *Agrobacterium* is suspended.

Also, since the infection of the *Agrobacterium* occurs in a wound of the plant tissue, when a shoot in which a wound is formed on its base part is soaked in an *Agrobacterium* suspension, the wound is infected with the *Agrobacterium* and the desired gene is introduced into the base part of the shoot. A hypocotyl is used as the tissue for gene introduction by cutting off roots and the like and the infection occurs at the end face (cut surface) of the base part of the hypocotyl formed by the cutting. That is, in this case, the desired gene is introduced into the base part of the hypocotyl by merely soaking the tissue for gene introduction simply in the *Agrobacterium* suspension.

Furthermore, a plant tissue is surely infected with the *Agrobacterium* when a tissue for gene introduction is soaked in the cell suspension and then cocultured with the *Agrobacterium* for several days by introducing the tissue on a solid medium. As the coculturing medium, a well known basal medium such as MS (Murashige and Skoog, Physiol. Plant, 15: 473-497 (1962)) or WPM (Loyd and McCown, Prop. Int. Plant Prop. Soc., 30: 421-427 (1980)), or a modified composition thereof to suit for the tissue for gene introduction to be infected with the *Agrobacterium,* can be used by supplementing it with a carbon source and a medium solidifying agent and, if necessary, with plant hormones such as auxins, cytokinins and the like appropriately. In this case, generally, 10 to 30 g/l sucrose is used as the carbon source; 5 to 10 g/l agar or 1 to 4 g/l gelan gum is used as the medium solidifying agent; 0.01 to 5.0 mg/l zeatin, benzyladenine or the like is used as the cytokinins; and 0.01 to 2.0 mg/l naphthaleneacetic acid (NAA), indolebutyric acid (IBA), indoleacetic acid or the like is used as the auxins. Also, infectivity of the *Agrobacterium* is increased in some cases by adding 10 to 200 mg/l acetosyringon to the above coculturing medium.

On the other hand, when a desired gene is introduced by a particle gun, the above coculturing medium can be used as the medium for gene introduction treatment. In this case, a tissue for gene introduction is placed on the medium with its position into which the desired gene is to be introduced, namely the base part of the hypocotyl, upside, and the gene introduction is carried out by manipulating the particle gun in the usual way.

In general, when a desired gene is introduced into a tissue for gene introduction, a selectable marker gene is introduced together with the desired gene, and expression of the selectable marker gene is used as an index of the introduction of the desired gene. In the method of the present invention, the transformation efficiency can be further improved by using a cytokinin-related gene as the selectable marker gene.

Herein, the cytokinin-related gene is a gene which acts in a direction of increasing the influence of cytokinin in the introduced plant cells and thereby increases adventitious bud differentiation ability of the cells. Examples of the gene include the *ipt* gene as an *A*. *tumefaciens*-derived cytokinin synthesis gene (A.C. Smigocki and L.D. Owens, Proc. Natl. Acad. Sci. USA, 85: 5131 (1988)), the *Escherichia coli*-derived β-glucuronidase gene as a gene which activates inactive cytokinin (Morten Loersbo and Finn T. Okkels, Plant Cell Reports, 16: 219-221 (1996)), the *Arabidopsis thaliana-*derived *CK11* gene which is considered to be a cytokinin receptor gene (Kakimoto T., Science, 274: 982-985 (1996)) and the like. Particularly, the *ipt* gene used in Examples of the present specification is a gene which is most well known and whose function has been revealed.

Also, the desired gene, the cytokinin-related gene and other nucleotide sequences and genes which are optionally introduced may be introduced by inserting them into the same vector or be introduced by inserting them into different vectors with no problems, so long as they are incorporated into the same cells of the tissue for gene introduction. However, when the genes and the like are incorporated into the same vector, it is necessary to arrange them such that the presence of one side of genes and the like does not inhibit expression of the other side of genes and the like.

An adventitious bud can be differentiated from a tissue after the gene introduction by culturing the tissue using an appropriate medium. A composition of the medium suitable for the adventitious bud differentiation varies depending on each plant, but in the case of the genus *Eucalyptus,* the MS medium in which the concentration ratio of ammonia nitrogen and nitrate nitrogen is changed to 1:3 (hereinafter simply referred to as "modified MS medium") can be used as the medium for adventitious bud differentiation (the shoot regeneration medium) after diluting it to 1 to 4 folds and supplementing it with 10 to 30 g/l sucrose, 1 to 4 g/l gelan gum or 5 to 10 g/l agar, and 0.2 to 5.0 mg/l zeatin and 0.01 to 1.0 mg/l NAA as plant hormones. However, when a cytokinin-related gene is used as the selectable marker gene, the plant hormones may not be added (may be hormone-free) or auxin alone may be added. Also, when a gene is introduced by the above *Agrobacterium* method, antibiotics such as carbenicillin, ticarcillin, cefotaxime and the like are added to the medium in an amount of 10 to 10,000 mg/l to inhibit the *Agrobacterium* growth. It is preferable that the temperature is from 15 to 30°C and the light intensity is from 0 to 200 µnol/m²/s. Since the apical bud or apical bud primordium of a hypocotyl preserved at the time of the gene introduction is not particularly required in the subsequent steps, it can be cut off at an appropriate stage.

The tissue cultured using the hypocotyl regeneration medium differentiation differentiates the adventitious bud generally several weeks after commencement of the culturing. In this case, a callus may grow slightly prior to the adventitious bud differentiation. The desired gene is introduced into the thus differentiated adventitious bud at higher frequency than the differentiated adventitious bud differentiated by introducing the gene into a segment based on the conventional method. However, when the gene introduction is carried out using a cytokinin-related gene as a selectable marker gene, the adventitious bud into which the desired gene has been introduced may sometimes show morphological abnormality such as multiple bud or the like due to the induction of morphological abnormality by the gene. Even in that case, however, an adventitious bud having normal morphology can finally be obtained by removing influence of the cytokinin-related gene when the cytokinin-related gene is used in combination with a DNA factor having leaving ability as shown, for example, in JP-A-9-154580.

A plantlet into which the desired gene has been introduced can be regenerated by cutting out the thus obtained adventitious bud and transplanting it on a rooting medium containing, for example, 0 to 1.0 mg/ml auxins, for rooting.

The present invention is based on the knowledge that a desired gene is efficiently introduced into cells of a base part of a hypocotyl, obtained by germinating a seed, having no growing point when the base part is subjected to a gene introduction treatment while retaining the apical bud or apical bud primordium of the hypocotyl, and that the base part of the hypocotyl into which the desired gene has been introduced in this manner has higher ability to redifferentiate an adventitious bud into which the desired gene has been introduced, in comparison with the case of introducing the desired gene into the base of the hypocotyl from which growing point has been removed.

The reason for this is not necessarily clear. However, it is considered that the apical bud or apical bud primordium which is present in the same hypocotyl is contributing to this in some forms. Since plant tissues and cells are always damaged at a certain degree in carrying out gene introduction and the recovering strength from this damage is reinforced by the presence of a growing point, it seems that active growth ability is also maintained in the gene-introduced cells, and thereby acts advantageously on the introduction of the desired gene, and/or redifferentiation of an adventitious bud into which the desired gene has been introduced.

In addition, the base part of the hypocotyl having no growing point is generally considered to be a part suitable for rooting. Accordingly, it is considered that when a gene is introduced into the part using a cytokinin-related gene as the selectable marker gene, difference in the ability differentiating an adventitious bud becomes sharply large between the gene-introduced cells and not-introduced cells and, as a result, the cells into which the desired gene has been introduced selectively differentiate adventitious buds. Thus, the use of such a gene as the selectable marker gene more advantageously results in the differentiation of the adventitious bud into which the desired gene has been introduced.

According to the present invention, introduction efficiency of a desired gene can be improved by the method for introducing a gene into a plant. Furthermore, in the present invention, an adventitious bud into which a desired gene has been introduced are differentiated efficiently from a tissue introduced with the desired gene. Thus, according to the present invention, introduction efficiency of the desired gene into an adventitious bud is particularly improved.

The present invention can be applied to many plants, and the effect of the present invention has a great meaning particularly for a plant in which gene introduction has been considered to be difficult.

That is, according to the present invention, gene introduction into industrially important tree species can be carried out and the present invention opens a way for preparing a transformant which can be practically valued.

The present invention is explained below based on Examples in details; however, the present invention is not limited thereto.

### Example 1

Seeds of *Eucalyptus camaldulensis* (hereinafter referred to as "*E. camaldulensis*") were sterilized by soaking them in 70% ethanol for 1 minute and further soaking in 2% aqueous sodium hypochlorite solution for about 2 hours with stirring, washed thoroughly with sterile water and inoculated onto a germination medium, preserved for 2 days or more in a refrigerator of 4°C for accelerating germination, and then cultured at 25°C under whole light condition of 40 µol/m²/s in light intensity to effect their germination. In this case, a 2-fold diluted modified MS medium (hereinafter referred to as "*camaldulensis* basal medium") was supplemented with 10 g/l sucrose and 8 g/l agar and used as the germination medium.

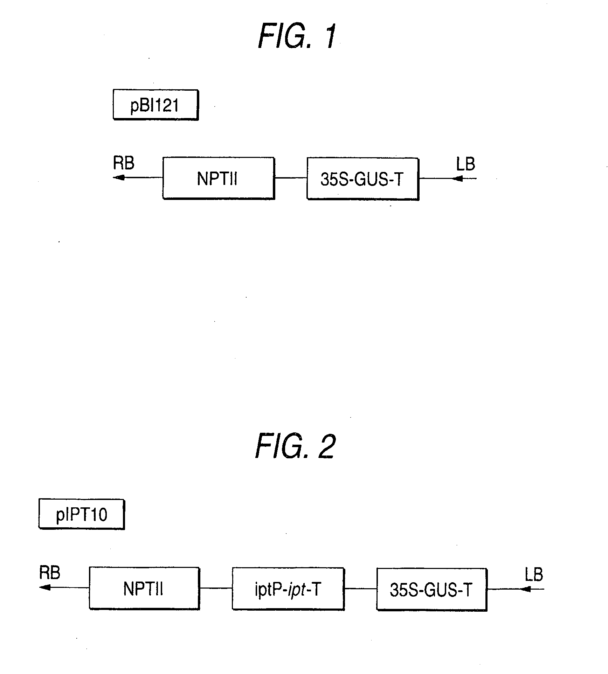

One to two weeks after the inoculation of seeds onto the germination medium, roots and seed leaves were cut off from the thus germinated seedlings to collect each hypocotyl keeping back apical bud alone on its one end, and a pBI121 vector (available from CLONTECH, cf. Fig. 1) was introduced into its base part having no growing point. In Fig. 1, NPTII indicates a kanamycin-resistant gene, and 35S-GUS-T indicates a GUS gene to which a 35S promoter and a terminator are connected at the 5' side and at the 3' side, respectively. That is, the pBI121 vector was introduced into *A. tumefaciens* EHA105 in advance by electroporation (using GENE PULSER II manufactured by Bio-Rad), followed by culturing overnight in YEB liquid medium and diluted to OD₆₃₀ = 0.5 with the *camaldulensis* basal medium to prepare a cell suspension, and then the hypocotyl collected in the above manner was soaked in the cell suspension. Next, after discarding excess cell suspension, the hypocotyl was cocultured with the *Agrobacterium* at 25°C for 2 days in the dark using the shoot regeneration medium supplemented with 40 mg/l acetosyringon, to thereby infect it with the pBI121 vector-introduced *Agrobacterium.* In this case, the *camaldulensis* basal medium was supplemented with 2.0 mg/l zeatin, 0.3 mg/l NAA, 10 g/l sucrose and 8 g/l agar and used as the shoot regeneration medium.

The pBI121 vector used herein is a vector prepared by inserting the GUS gene as a model of the desired gene and a kanamycin-resistant gene (NPTII gene) as the selectable marker gene. Thus, the cells are introduced with the GUS gene and kanamycin-resistant gene by the introduction of the pBI121 vector, and show GUS activity and kanamycin resistance.

The hypocotyl after coculturing was transplanted onto the shoot regeneration medium further supplemented with 500 mg/l ticarcillin and 50 mg/l kanamycin for the selection of pBI121 vector-introduced cells and cultured at 25°C under whole light conditions of 40 µmol/m²/s in light intensity by sub-culturing it using the same medium composition at an interval of 2 weeks, and 3 months after the *Agrobacterium* infection, the thus differentiated and elongated adventitious bud was rooted using a rooting medium. As the rooting medium, the *Eucalyptus* basal medium was used after supplementing it with 0.05 mg/l IBA, 500 mg/l ticarcillin, 150 mg/l kanamycin, 10 g/l sucrose and 2.5 g/l gelan gum.

When the experimentation was repeated three times on 50 hypocotyls, differentiation of adventitious buds was started to be observed 6 weeks after the *Agrobacterium* infection in each case, and finally, rooting from buds originated from 6 of the 150 hypocotyls (hereinafter referred to as "buds of 6 lines") was observed after 4 months. When a GUS activity test was carried out on the buds in accordance with the method of Jefferson *et al.,* its expression was confirmed in buds of 5 lines. That is, the transformation efficiency in this case was 6/150 × 100 = 4.0% based on the kanamycin resistance and 5/150 × 100 = 3.3% based on the GUS activity. The results are shown in Table 1.

### Comparative Example 1

Introduction of the GUS gene and kanamycin-resistant gene and subsequent regeneration of plants were carried out in the same manner as in Example 1, except that those which were prepared by cutting off roots, cotyledons and apical buds from the germinated seedlings of *E. camaldulensis* seeds were used as the hypocotyls for gene introduction.

As a result of the test on 320 hypocotyls, rooting was observed finally in buds of 8 lines, and the GUS activity was observed in buds of 6 lines among them. That is, the transformation efficiency in this case was 8/320 × 100 = 2.5% based on the kanamycin resistance and 6/320 × 100 = 1.9% based on the GUS activity. The results are shown in Table 1.

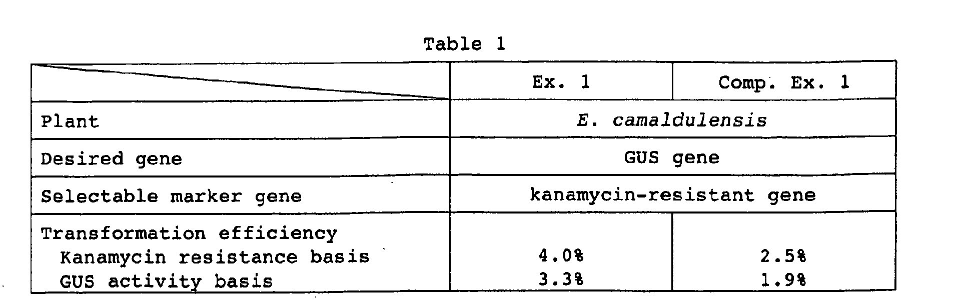

### Example 2

Instead of the kanamycin-resistant gene of pBI121 vector used in Example 1, a pIPT10 vector having *A. tumefaciens* PO22-derived *ipt* gene (its preparation method is described in JP-A-11-197722, cf. Fig. 2) was introduced as the selectable marker gene into the hypocotyl base of *E. camaldulensis* prepared by cutting off roots and seed leaves and keeping only the apical bud on its one end, and adventitious bud was differentiated in the same manner as in Example 1. In Fig. 2, iptP-*ipt*-T indicates the *ipt* gene to which the promoter of the *ipt* gene itself and a terminator are connected at the 5' side and at the 3' side, respectively, and the others are the same as in Fig. 1. In this case, however, the hypocotyl was precultured for 1 day using the shoot regeneration medium prior to infection with *Agrobacterium.* Also, plant hormone and kanamycin were not added to the medium for adventitious bud differentiation after the *Agrobacterium* infection. Apical buds of hypocotyls were cut off after 10 weeks from the *Agrobacterium* infection.

When tests were carried out using 40 hypocotyls, growth of some calli was observed prior to the differentiation of adventitious buds in this case. Accordingly, the GUS activity test was carried out on the adventitious buds differentiated from calli and also on the calli themselves, 3 months after the *Agrobacterium* infection. As a result, the GUS activity was found in calli derived from 20 hypocotyls (hereinafter referred to as "calli of 20 lines"), 6 lines among the calli of 20 lines differentiated adventitious buds, and adventitious buds of 4 lines among them showed the GUS activity. That is, on the GUS activity basis, the transformation efficiency into calli was 20/40 × 100 = 50.0%, and the transformation efficiency into adventitious buds was 4/40 × 100 = 10.0%. The results are shown in Table 2.

### Comparative Example 2

Adventitious buds were differentiated by introducing the GUS gene and the *ipt* gene using the pIPT10 vector in the same manner as in Example 2, except that those in which roots, cotyledons and apical buds were cut off from germinated seedlings of *E. camaldulensis* seeds were used as the hypocotyls for gene introduction.

When tests were carried out using 98 hypocotyls, growth of some calli was observed prior to the differentiation of adventitious buds in this case, too. As a result of the GUS activity test 3 months after the *Agrobacterium* infection, the GUS activity was found in calli of 40 lines, and 5 lines among the calli of 40 lines differentiated adventitious buds. However, the GUS activity was not able to be found in any of the adventitious buds. That is, on the GUS activity basis, the transformation efficiency into calli was 40/98 × 100 = 40.8%, and the transformation efficiency into adventitious buds was 0/98 × 100 = 0.0%. The results are shown in Table 2.

### Example 3

Seeds of *Eucalyptus globulus* (hereinafter referred to as "*E. globulus*") were sterilized by soaking them in 70% ethanol for 1 minute and further soaking in 2% aqueous sodium hypochlorite solution for about 4 hours with stirring, washed thoroughly with sterile water and inoculated onto a germination medium, preserved for 2 days or more in a refrigerator of 4°C for accelerating germination, and then cultured at 25°C under whole light conditions of 40 µol/m²/s in light intensity for the germination. In this case, the MS medium was supplemented with 0.5 mg/l zeatin, 20 g/l sucrose and 9 g/l agar and used as the germination medium.

One to two weeks after the inoculation of seeds onto the germination medium, roots and cotyledons were cut off from the thus germinated seedlings to obtain the hypocotyls each keeping apical bud alone on its one end, and the GUS gene was introduced into its base part having no growing point, together with the kanamycin-resistant gene as a selectable marker gene. That is, the hypocotyl was soaked in a pBI121 vector (available from CLONTECH, cf. Fig. 1)-introduced *Agrobacterium* cell suspension prepared in the same manner as in Example 1, and after discarding excess cell suspension, cocultured with the *Agrobacterium* at 25°C for 3 days in the dark using a medium for adventitious bud differentiation supplemented with 40 mg/l acetosyringon, to thereby infect it with the pBI121 vector-introduced *Agrobacterium.* In this case, the concentration of the nitrogen source alone in the modified MS medium was changed to 1/2, and the resulting medium was supplemented with 1.0 mg/l zeatin, 0.05 mg/l NAA, 20 g/l sucrose and 9 g/l agar and used as the medium for adventitious bud differentiation.

The hypocotyl after coculturing was transplanted onto the medium for adventitious bud differentiation further supplemented with 500 mg/l ticarcillin and 100 mg/l kanamycin for the selection of pBI121 vector-introduced cells and cultured at 25°C under whole light conditions of about 40 µmol/m²/s in light intensity by sub-culturing it using the same medium composition at an interval of 2 weeks, to thereby differentiate adventitious buds. In this case, the apical bud of hypocotyl was cut off after 1 month of the *Agrobacterium* infection.

When tests were carried out on 182 hypocotyls, growth of some calli was observed prior to the differentiation of adventitious buds in this case, too. Accordingly, the GUS activity test was carried out on the adventitious buds differentiated from calli and also on the calli themselves, 3 months after the *Agrobacterium* infection. As a result, the GUS activity was found in calli of 40 lines, 12 lines among the calli of 40 lines differentiated adventitious buds, and adventitious buds of 6 lines among them showed the GUS activity. That is, on the GUS activity basis, the transformation efficiency into calli was 40/182 × 100 = 22.0%, and the transformation efficiency into adventitious buds was 6/182 × 100 = 3.3%. The results are shown in Table 2.

### Comparative Example 3

Introduction of the GUS gene and kanamycin-resistant gene and subsequent regeneration of plants were carried out in the same manner as in Example 3, except that those which were prepared by cutting off roots, seed leaves and apical buds from the germinated seedlings of *E. globulus* seeds were used as the hypocotyls for gene introduction.

When tests were carried out on 220 hypocotyls, growth of some calli was observed prior to the differentiation of adventitious buds in this case, too. As a result of the GUS activity test carried out 3 months after the *Agrobacterium* infection, the GUS activity was found in calli of 9 lines, 2 lines among the calli of 9 lines differentiated adventitious buds, and the adventitious bud of 1 line among them showed the GUS activity. That is, on the GUS activity basis, the transformation efficiency into calli was 9/220 × 100 = 4.1%, and the transformation efficiency into adventitious buds was 1/220 × 100 = 0.5%. The results are shown in Table 2.

### Example 4

The pIPT10 vector (its preparation method is described in JP-A-11-197722, cf. Fig. 2) was introduced into the hypocotyl base of *E. globulus* prepared by cutting off roots and seed leaves and keeping only the apical bud on its one end, and adventitious bud was differentiated in the same manner as in Example 3. In this case, however, plant hormone and kanamycin were not added to the medium for adventitious bud differentiation.

As a result of tests on 120 hypocotyls, adventitious buds were differentiated from 22 hypocotyls until 4 months after their infection with *Agrobacterium,* and the GUS activity was found in the adventitious buds of 8 lines among them. That is, the transformation efficiency into adventitious buds in this case was 8/120 × 100 = 6.7% based on the GUS activity. The results are shown in Table 2.

### Comparative Example 4

Adventitious buds were differentiated by introducing the GUS gene and the *ipt* gene using the pIPT10 vector in the same manner as in Example 4, except that those which were prepared by cutting off roots, seed leaves and apical buds from the germinated seedlings of *E. globulus* seeds were used as the hypocotyls for gene introduction.

As a result of tests on 180 hypocotyls, adventitious buds were differentiated from 18 hypocotyls until 4 months after the infection with *Agrobacterium,* and the GUS activity was found in the adventitious buds of 6 lines among them. That is, the transformation efficiency into adventitious buds in this case was 6/180 × 100 = 3.3% based on the GUS activity. The results are shown in Table 2.

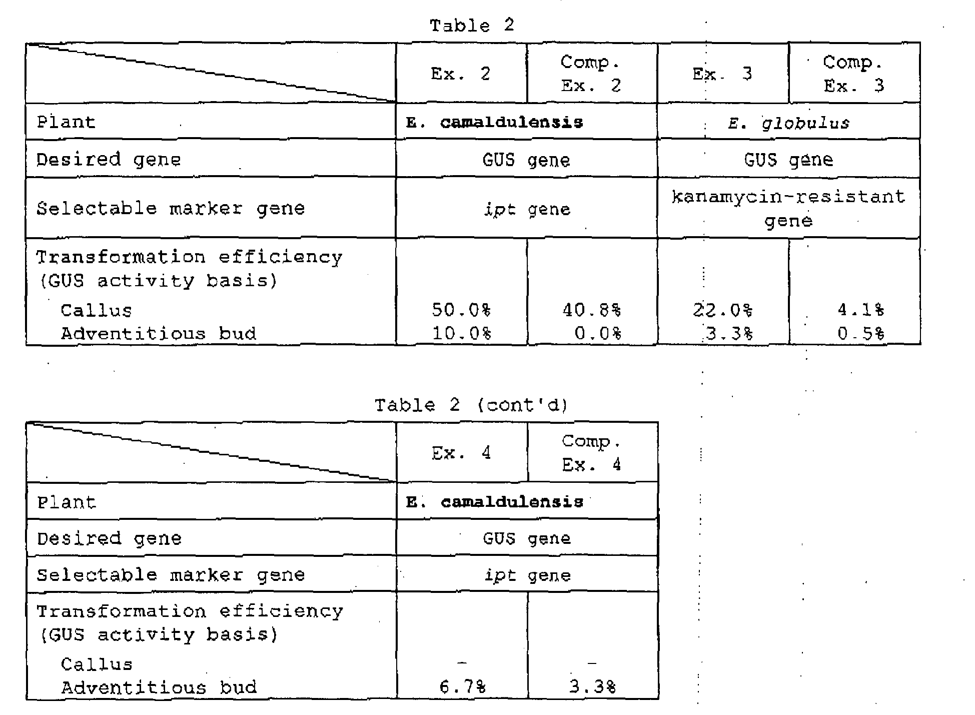

As is apparent from Tables 1 and 2, all of the Examples 1 to 4 showed higher transformation efficiency on the kanamycin resistance basis and GUS gene expression basis than the respective corresponding Comparative Examples 1 to 4.

Also, the GUS gene-introduced calli in Examples 2 and 3 showed higher probability to differentiate GUS gene-introduced adventitious buds than those in Comparative Examples 2 and 3. Furthermore, the probability in Example 2 is 4/20 × 100 = 20.0% because GUS gene-introduced adventitious buds were differentiated from 4 lines among 20 lines of the GUS gene-introduced calli, the probability in Comparative Example 2 is 0/40 × 100 = 0% because 40 lines of GUS gene-introduced calli were obtained but all of them did not differentiate GUS gene-introduced adventitious bud, the probability in Example 3 is 6/40 × 100 = 15.0% because GUS gene-introduced adventitious buds were differentiated from 6 lines among 40 lines of the GUS gene-introduced calli, and the probability in Comparative Example 3 is 1/9 × 100 = 11.1% because a GUS gene-introduced adventitious bud was differentiated from 1 line among 9 lines of the GUS gene-introduced calli.

As the reason for this, it is considered that the GUS gene-introduced calli in Examples 2 and 3 actively differentiated adventitious buds as a whole in comparison with the GUS gene-introduced calli in Comparative Examples 2 and 3. The adventitious bud differentiation ratio from the GUS gene-introduced calli supports the reason. That is, the adventitious bud differentiation ratio in these cases was 6/20 × 100 = 30% in Example 2, 5/40 × 100 = 12.5% in Comparative Example 2, 12/40 × 100 = 30% in Example 3 and 2/9 × 100 = 22.2% in Comparative Example 3. It is considered that such an improvement in the adventitious bud differentiation ratio is due to an influence of the apical bud kept on one end of the hypocotyl used as a tissue for gene introduction in Examples 2 and 3.

While the invention has been described in detail and with reference to specific examples thereof, it will be apparent to one skilled in the art that various changes and modifications can be made therein without departing from the spirit and scope thereof. All references cited herein are incorporated in their entirety.

The priority application is Japanese patent application No. 2001-223664, filed July 24, 2001.

## Claims

1. A method for producing a transgenic plant, which comprises:
preparing a tissue for gene introduction by cutting off a root and a root base from a hypocotyl, obtained by germinating a seed, having an apical bud or an apical bud primordium, to thereby form an end face whilst retaining the apical bud or the apical bud primordium; and
introducing a desired gene into the base part of the hypocotyl, to thereby differentiate an adventitious bud.

2. A method according to claim 1, wherein the hypocotyl is a hypocotyl of the genus *Eucalyptus.*

3. A method according to claim 1 or 2, wherein the desired gene is introduced into the end face.

4. A method according to any preceding claim, wherein the desired gene is introduced using an *Agrobacterium* method.

5. A method according to any preceding claim, wherein a cytokinin-related gene is introduced as a selectable marker gene together with the desired gene.

6. A method according to claim 5, wherein the cytokinin-related gene is the *ipt* (isopentenyl transferase) gene.

## Patentansprüche

1. Verfahren zur Herstellung einer transgenen Pflanze, umfassend:
Vorbereiten eines Gewebes zur Geneinführung, indem ein Stamm und eine Stammbasis von einem Hypokotyl, das durch Keimen eines Saatkorns erhalten wird, und eine Gipfelknospe oder Gipfelknospenprimordium aufweist, abgeschnitten wird, um so eine Endfläche zu bilden, während die Gipfelknospe oder das Gipfelknospenprimordium bewahrt wird; und
Einführen eines gewünschten Gens in den Basisteil des Hypokotyls, um so eine adventive Knospe zu differenzieren.

2. Verfahren nach Anspruch 1, wobei das Hypokotyl ein Hypokotyl der Art *Eucalyptus* ist.

3. Verfahren nach Anspruch 1 oder 2, wobei das gewünschte Gen in die Endfläche eingeführt wird.

4. Verfahren nach einem der vorhergehenden Ansprüche, wobei das gewünschte Gen unter Anwendung eines *Agrobacterium*-Verfahrens eingeführt wird.

5. Verfahren nach einem der vorhergehenden Ansprüche, wobei ein cytokininverwandtes Gen als auswählbares Marker-Gen zusammen mit dem gewünschten Gen eingeführt wird.

6. Verfahren nach Anspruch 5, wobei das cytokinin-verwandte Gen das *ipt-*(Isopentenyl-Transferase)-Gen ist.

## Revendications

1. Procédé pour produire une plante transgénique, qui consiste à :
préparer un tissu pour l'introduction d'un gène en coupant une racine et une base de racine issue d'un hypocotyle, obtenu en faisant germer une graine, ayant un bourgeon apical ou un primordium de bourgeon apical, afin d'ainsi former une face terminale tout en conservant le bourgeon apical ou le primordium de bourgeon apical ; et
introduire un gène souhaité dans la partie de base de l'hypocotyle, afin d'ainsi différencier un bourgeon adventif.

2. Procédé selon la revendication 1, dans lequel l'hypocotyle est un hypocotyle du genre *Eucalyptus.*

3. Procédé selon la revendication 1 ou 2, dans lequel le gène souhaité est introduit dans la face terminale.

4. Procédé selon l'une quelconque des revendications précédentes, dans lequel le gène souhaité est introduit en utilisant un procédé avec *Agrobacterium.*

5. Procédé selon l'une quelconque des revendications précédentes, dans lequel un gène associé à une cytokinine est introduit en tant que marqueur sélectionnable avec le gène souhaité.

6. Procédé selon la revendication 5, dans lequel le gène associé à une cytokinine est le gène *ipt* (isopentényl transférase).
